# EUROPEAN PATENT SPECIFICATION

(11) **EP 1 720 481 B1**
(45) Date of publication and mention of the grant of the patent: **13.10.2010**
(21) Application number: 05726523.3
(22) Date of filing: 20.01.2005
(51) Int. Cl.: A61C 8/00

(54) **Anchoring element**
Verankerungselement
Elément d'ancrage

(30) Priority: 02.02.2004 SE 0400230
(43) Date of publication of application: 15.11.2006
(73) Proprietor: Brånemark Integration AB, 411 04 Göteborg (SE)
(72) Inventor: ÖHRNELL, Lars-Olof, S-412 74 Göteborg (SE); BRÅNEMARK, Rickard, S-431 38 MÖLNDAL (SE); MORENIUS, Carl, S-431 62 MÖLNDAL (SE); NILSSON, Peter, S-566 92 HABO (SE); SVENSSON, Thomas, S-416 74 GÖTEBORG (SE)
(74) Representative: Hopfgarten, Nils
(86) International application number: PCT/SE2005/000056
(87) International publication number: WO 2005/079697

(56) References cited:
- EP-A2- 0 599 794
- US-A- 4 463 753
- US-A- 5 324 199
- US-B1- 6 413 089

## Description

### Technical Field

The present invention relates to an elongated anchoring element according to the preamble of claim 1.

### Background of the Invention

Upon treatment of patients with different bone fractures, the use of various types of fixation means has been known for a long time, which are implanted in the patient's tissue, for example in order to join different bone tissues, which completely or partly have been separated through bone fractures. It is essential to choose a material and a design of these fixation means so that the body does not reject these fixation means, that inflammations do not occur, and that the fixation means are properly anchored in the bone tissue.

Various types of fixation means are implanted in patients' jaws, for example for patients with highly resorbed bone or very poor bone quality in the maxilla, i.e. the upper jaw. For these patients a double-anchoring fixation means is used, e.g., which is anchored both in the maxilla and in the os zygomaticum, i.e. the zygomatic bone, which is located above the prominence of respective cheekbone towards respective ear. This double-anchoring fixation means is anchored subsequent to drilling, during which drilling the maxilla is drilled through so that a through hole is formed, after which the drilling is continued through the sinus maxillaris, i.e. the maxillary sinus, and is stopped up in the os zygomaticum. Usually, the os zygomaticum always has good bone quality, which guarantees a good result. The alterative to this treatment of patients with above mentioned bone status is to take a piece of bone from another part of the body, shape it and place it in the sinus maxillaries, to get a better support for the fixation means. Bone transplant involves a more expensive operation on a patient and a longer heading process.

EP 0 599 794 discloses a substantially cylindrical anchoring element for replacement of one or more teeth in the upper jaw, presenting two cylindrical portions having different diameters, which are provided with external threads having the same pitch. At one end the anchoring element has a mounting portion for the mounting of a denture or a dental bridge. The two cylindrical portions are intended for fixation in the zygomatic bone and the upper jaw, respectively.

However, the anchoring element disclosed in EP 0 599 794, and other double-anchoring fixation means according to prior art, have several disadvantages. Since it is important that a double-anchoring fixation element in the jaws is stable and firmly anchored, it is, besides a proper fixation in the os zygomaticum, essential that the fixation element is also firmly anchored in the maxilla. This is problematic since the maxilla of patients with poor bone quality is often very thin. If there is a poor fixation in the maxilla, there is a major risk of inflammations and other complications. Prior fixation elements do not completely succeed in solving this problem. Further, the fixation in the os zygomaticum of prior fixation elements is not strong enough. There is also a need for an effective double-anchoring fixation element upon other types of treatments, for example when two bones at another location in the body are to be joined after a complicated fracture. Fixation elements according to prior art neither solves the problem when the maxilla is completely or partly removed, for example as a consequence of cancer in the jaw region, and a dental bridge or a denture is to be arranged at the original position of the maxilla, and the only possibility of fixation is in the os zygomaticum. There is also a need for an effective single-anchoring fixation element for treatments at other locations in a patient's body.

### The Object of the Invention

The object of the present invention is therefore to provide an anchoring element which improves and facilitates the fixation in the maxilla when a fixation shall be made in both the maxilla and the os zygomaticum, especially on patients with poor bone quality which improves the fixation in the os zygomaticum.

### Summary of the Invention

The above mentioned objects of the present invention are attained by providing an anchoring element of the kind defiled in the preamble of claim 1, where the second fixation portion has the form of a truncated cone having the smaller basis adjacent to the intermediate portion and the larger basis forming the basis of the anchoring element. This conic shape of the second fixation portion provides for the anchoring element to be automatically wedged in the bore in the second bone tissue, and simultaneously provides for the apex of the anchoring element to be forced into the bore in the first bone tissue. The anchoring element is advantageously positioned in a patient's jaw, e.g., the fist bone tissue being located in the os zygomaticum, and the second bone tissue being located in the maxilla, and an attachment means intended for a denture or a dental bridge is arranged at the basis of the anchoring element. Advantageously, the attachment means comprises an attachment hole, which for example presents inner threads, and at least one contact surface for the denture or the dental bridge, and the attachment hole is arranged at an angle to the longitudinal axis of the anchoring element.

According to an advantageous embodiment of the anchoring element according to the present invention, also the first fixation portion has the form of a truncated cone having the smaller basis adjacent to the apex and the larger basis adjacent to the intermediate portion. In this way the fixation in the first bone tissue becomes more effective, Inter alia for reasons of facilitating the production, it Is also advantageous that the first and second fixation portions and the intermediate portion together have the form of a truncated cone having the smaller basis adjacent to the apex and the larger basis forming the basis of the anchoring element.

According to a further advantageous embodiment of the anchoring element according to the present invention, the first fixation portion is threaded. In this way a firm fixation of the anchoring element in the first bone tissue is achieved when it is driven in by rotation. Advantageously, also the second fixation portion is threaded. An alternative to a threaded fixation portion is to provide the fixation portion with a rough surface, for example, which is attached to the bone tissue through pressure.

According to another advantageous embodiment of the anchoring element according to the present invention, the second fixation portion comprises at least one recess transverse to the threads and disposed at the intermediate portion, comprising a distinct edge transverse to the threads, which edge provides a groove forming cutting unit. Advantageously, there are several recesses, for example three, which are evenly distributed along the circumference. In this way, the second fixation portion becomes self-tapping since grooves are cut on the inside of the bore in the second bone tissue through the rotation of the anchoring element, in which grooves the threads of the second fixation portion subsequently engage. In order to avoid inflammations in non-bone tissue between the first and second bone tissues, the intermediate portion advantageously has a smooth outer surface. Sometimes the anchoring element is diagonally positioned to such an extent that it is positioned outside the maxilla, where there are, inter alia, masticatory muscles which could be Irritated by the threads, and therefore the smooth outer surface is advantageous. If the intermediate portion is smooth and, in addition, the fixation portions are threaded, it is advantageous that the outer surface of the intermediate portion, the surface limited by the valleys of the threads of the threaded first fixation portion, and the surface limited by the valleys of the threads of the threaded second fixation portion are portions of the envelope surface of the same truncated cone.

### Brief Description of the Drawings

The present invention will now be described, for exemplary purposes, in more detail by way of embodiments and with reference to the endorsed drawings, in which:
- Fig. 1: is a side view of an embodiment of the anchoring element accord- ing to the present invention.
- Fig. 2: is a side view of an embodiment of the dental anchoring member according to the present invention,
- Fig. 3: is a side view of an embodiment of the dental anchoring unit according to the present invention.
- Fig. 4: is a schematic view of an example of the position of the anchoring element or dental anchoring member according to the present in- vention, in relation to the os zygomaticum and the maxilla, and
- Fig. 5: is a schematic view of an example of the fixation of an embodi- ment of the anchoring element or dental anchoring member of the present invention in the os zygomaticum and the maxilla.

### Detailed Description of the Embodiments

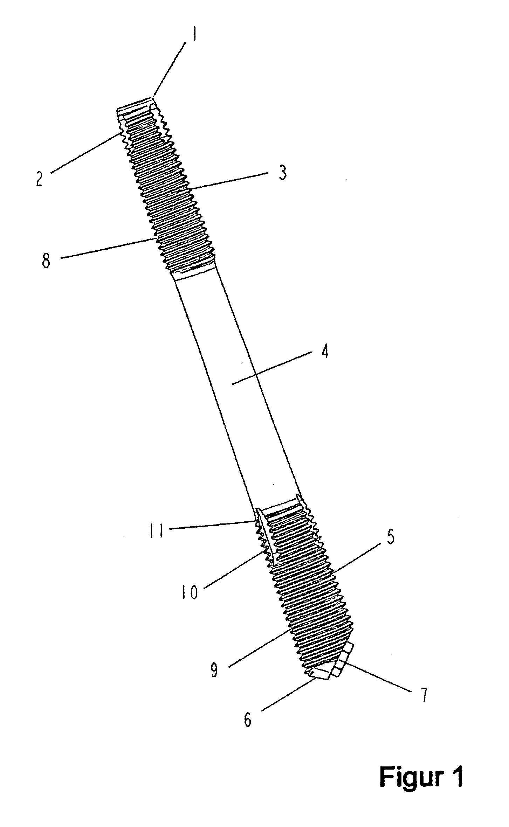

Fig. 1 shows an embodiment of the anchoring element according to the invention, for fixation in a first bone tissue and in a second bone tissue, the first and second bone tissues being separated by non-bone tissue. The anchoring element comprises an apex 1 according to prior art, provided with elongated recesses 2 which function as cutting units when the apex 1 is screwed into the first bone tissue and thus form grooves in which the threads engage. Adjacent to the apex 1 there is a threaded first fixation portion 3 which together with the apex 1 is adapted for fixation in the first bone tissue, and has the form of a truncated cone having the smaller basis adjacent to the apex 1 and the larger basis adjacent to an intermediate portion 4. Adjacent to the intermediate portion 4 there is a second fixation portion 5 adapted for fixation in the second bone tissue, and has the form a truncated cone having the smaller basis adjacent to the intermediate portion 4 and the larger basis forming the basis 6 of the anchoring element. At the basis 6 of the anchoring element, an attachment means 7 is arranged, intended for a denture or a dental bridge. The attachment means 7 advantageously comprises an attachment hole, which has inner threads, and two contact surfaces for the denture or the dental bridge. The attachment hole is arranged at an angle of about 45 ° to the longitudinal axis of the anchoring element. The outer surface of the intermediate portion 4, the surface limited by the valleys 8 of the threads of the threaded first fixation portion 3, and the surface limited by the valleys 9 of the threads of the threaded second fixation portion 5 are portions of the envelope surface of the same truncated cone. On the second fixation portion 5, there is a recess 10 transverse to the threads and disposed at the intermediate portion 4, comprising a distinct edge 11 transverse to the threads, which edge 11 provides a groove forming cutting unit. Two additional such recesses are evenly distributed along the circumference of the second fixation portion 5, but are not visible in Fig. 1. The smaller basis of the first fixation portion 3 has a diameter of 4 mm, e.g., and the larger basis of the second fixation portion 5 has a diameter of 5.3 mm, e.g.. The length of the first fixation portion 3 is 14 mm, e.g., and the length of the second fixation portion is 12 mm, e.g. The total length from the top of the apex 1 to the larger basis of the second fixation portion 5 is 36, 40, 44, 48 or 52 mm, e.g. By choosing the total length, a certain slope of the cone shape is provided.

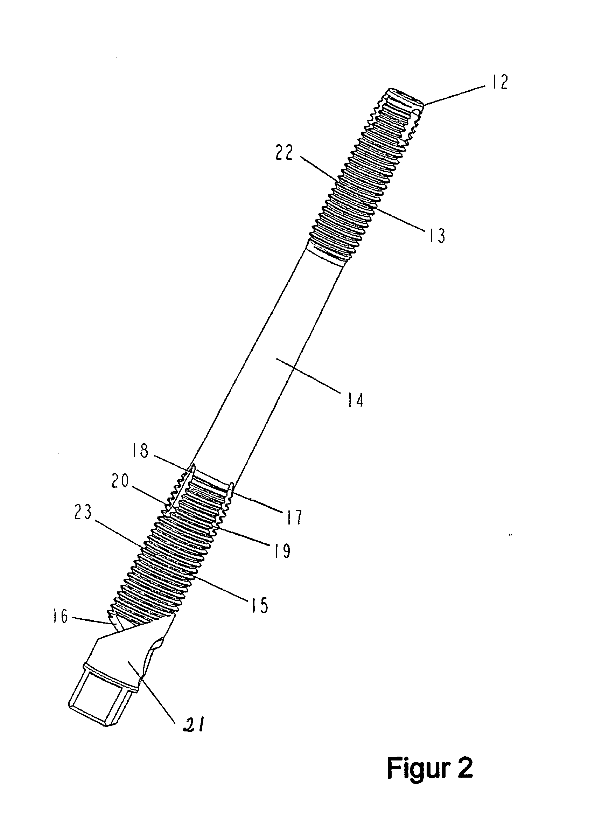

Fig. 2 shows an embodiment of the dental anchoring member according to the invention for fixation in the os zygomaticum and the maxilla, the os zygomaticum and the maxilla being separated by non-bone tissue. The dental anchoring member is elongated and comprises an apex 12 according to prior art, as described in connection to Fig. 1, and a threaded first fixation portion 13 disposed at the apex 12, both adapted for fixation in os zygomaticum. The first fixation portion 13 has the form of a truncated cone having the smaller basis adjacent to the apex 12 and the larger basis adjacent to a smooth intermediate portion 14. The intermediate portion 14 is positionable in the non-bone tissue separating the os zygomaticum and the maxilla. A threaded second fixation portion 15 is disposed at the basis 16 of the dental anchoring member and is adapted for fixation in the second bone tissue. The second fixation portion 15 has the form of a truncated cone having the smaller basis adjacent to the intermediate portion 14 and the larger basis forming the basis 16 of the dental anchoring member. The second fixation portion comprises three, of which only two are visible in Fig. 2, recesses 17, 18 transverse to the threads and disposed at the intermediate portion 14, each comprising a distinct edge 19, 20 transverse to the threads, respective edge 19, 20 providing a groove forming cutting unit in the bore of the maxilla. At the basis 16 of the dental anchoring member an attachment means intended for a denture or a dental bridge is arranged, but enclosed by an dental anchoring member holder 21 which is removed when the dental anchoring member is anchored. The outer surface of the intermediate portion 14, the surface limited by the valleys 22 of the threads of the threaded first fixation portion 13, and the surface limited by the valleys 23 of the threads of the threaded second fixation portion 15 are portions of the envelope surface of the same truncated cone. The dimensions of the dental anchoring member are for example the same as for the anchoring element of Fig. 1.

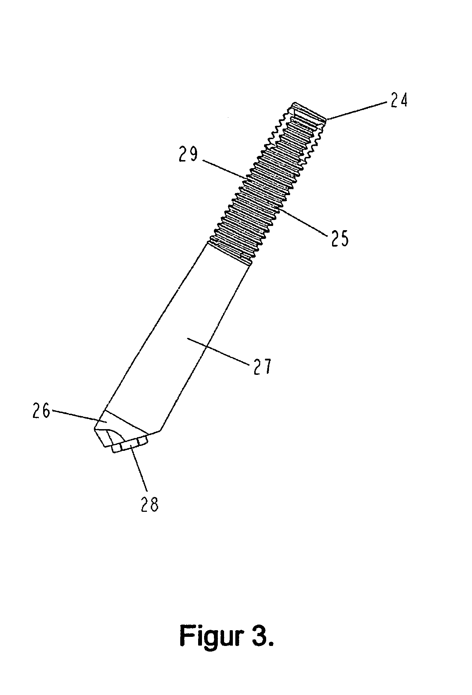

Fig. 3 shows an embodiment of the dental anchoring unit according to the invention for fixation in the os zygomaticum, the dental anchoring unit being elongated and comprising an apex 24 according to prior art, as described in connection to Fig. 1, and a threaded fixation portion 25 disposed at the apex 24, both adapted for fixation in the os zygomaticum, a basis 26, and an end portion 27 disposed between the fixation portion 25 and said basis 26. The fixation portion 25 and the end portion 27 together have the form of a truncated cone having the smaller basis adjacent to the apex 24 and the larger basis forming the basis 26 of the dental anchoring unit. At the basis 26 of the dental anchoring unit, an attachment means 28 intended for a denture or a dental bridge is arranged. The attachment means 28 comprises an attachment hole, which has inner threads, and two contact surfaces for the denture or the dental bridge. The attachment hole is arranged at an angle of about 45° to the longitudinal axis of the dental anchoring unit. The outer surface of the end portion 27 and the surface limited by the valleys 29 of the threads of the threaded fixation portion 25 are portions of the envelope surface of the same truncated cone.

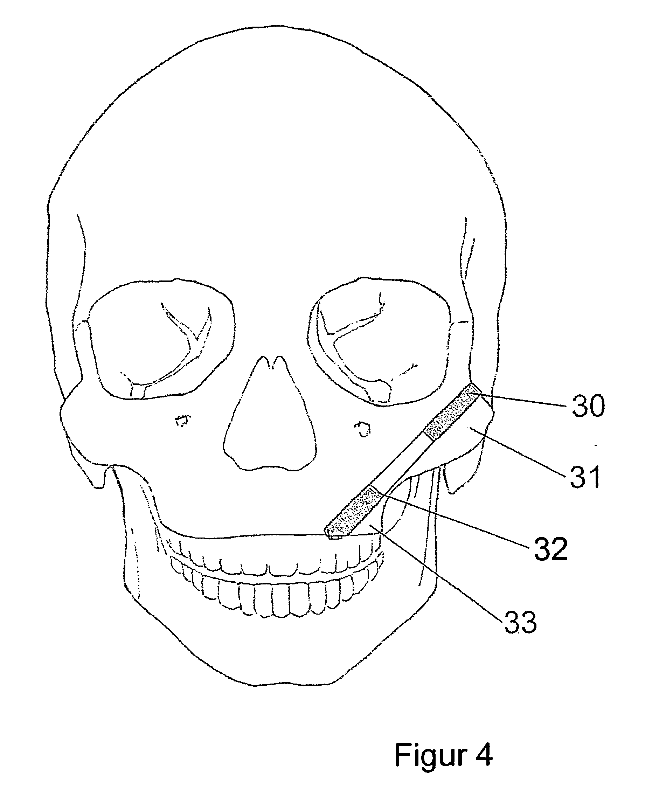

Fig. 4 shows an example of the position of the anchoring element or the dental anchoring member of the present invention in a patient's jaw, where the first fixation portion 30 is anchored in os zygomaticum 31, and the second fixation portion 32 is anchored in the maxilla 33.

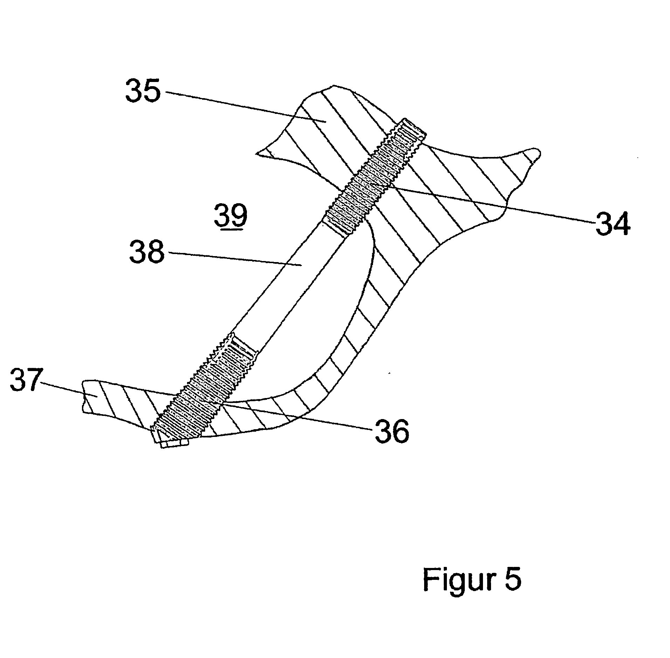

Fig. 5 shows a more precise example of the position of the anchoring element or the dental anchoring member of the present invention in a patient's jaw, where the first fixation portion 34 is anchored in os zygomaticum 35, and the second fixation portion 36 is anchored in the maxilla 37, and the smooth intermediate portion 38 is positioned in the sinus maxillaries 39.

## Claims

1. An anchoring element for fixation in a first bone tissue located in the os zygomaticum (31, 35) and in a second bone tissue located in the maxilla (33, 37), the first and second bone tissues being separated by non-bone tissue, the anchoring element being elongated and comprising an apex (1, 12) and a first fixation portion (3, 13, 30, 34) disposed at the apex (1, 12), both adapted for fixation in the first bone tissue, a basis (6, 16), a second fixation portion (5, 15, 32, 36) disposed at said basis (6, 16) and adapted for fixation in the second bone tissue, an intermediate portion (4, 14, 38) positionable in the non-bone tissue separating the first and second bone tissues, and attachment means (7), at the basis (6, 16) of the anchoring element, for attaching a denture or a dental bridge, **characterized in that** the second fixation portion (5, 15, 32, 36) has the form of a truncated cone having the smaller basis adjacent to the intermediate portion (4, 14, 38) and the larger basis forming the basis (6, 16) of the anchoring element.

2. An anchoring element according to any of the previous claims, **characterized in that** the first fixation portion (3,13, 30, 34) has the form of a truncated cone having the smaller basis adjacent to the apex (1, 12) and the larger basis adjacent to the intermediate portion (4, 14, 38).

3. An anchoring element according to any of the previous claims, **characterized in that** the first and second fixation portions (3, 13, 30, 34; 5, 15, 32, 36) and the intermediate portion (4, 14, 38) together have the form of a truncated cone having the smaller basis adjacent to the apex (1, 12) and the larger basis forming the basis (6, 16) of the anchoring element.

4. An anchoring element according to any of the previous claims, **characterized in that** the first fixation portion (3, 13, 30, 34) is threaded.

5. An anchoring element according to any of the previous claims, **characterized in that** the second fixation portion (5, 15, 32, 36) is threaded.

6. An anchoring element according to claim 5, **characterized in that** the second fixation portion (5, 15, 32, 36) comprises at least one recess (10, 17, 18) transverse to the threads and disposed at the intermediate portion (4, 14, 38), comprising a distinct edge (11, 19, 20) transverse to the threads, which edge (11, 19, 20) provides a groove forming cutting unit.

7. An anchoring element according to claim 5 or 6, **characterized in that** the intermediate portion (4, 14, 38) has a smooth outer surface.

8. An anchoring element according to claim 7, **characterized in that** the outer surface of the intermediate portion (4, 14, 38), the surface limited by the valleys (8, 22) of the threads of the threaded first fixation portion (3, 13, 30, 34), and the surface limited by the valleys (9, 23) of the threads of the threaded second fixation portion (5, 16, 32, 36) are portions of the envelope surface of the same truncated cone.

## Patentansprüche

1. Verankerungselement zur Fixierung in einem ersten Knochengewebe, welches sich im Os zygomaticum (31, 35) befindet, und in einem zweiten Knochengewebe, welches sich in der Maxilla (33, 37) befindet, wobei das erste und zweite Knochengewebe durch Nicht-Knochengewebe getrennt ist, das Verankerungselement länglich ist und einen Scheitel (1, 12) und einen ersten Fixierungsabschnitt (3, 13, 30, 34), der sich an dem Scheitel (1, 12) befindet, welche beide für eine Fixierung in dem ersten Knochengewebe geeignet sind, eine Basis (6, 16), einen zweiten Fixierungsabschnitt (5, 15, 32, 36), der sich an der Basis (6, 16) befindet und für eine Fixierung in dem zweiten Knochengewebe geeignet ist, einen Zwischenabschnitt (4, 14, 38), der in dem Nicht-Knochengewebe angeordnet werden kann, welches das erste und das zweite Knochengewebe trennt, und ein Befestigungsmittel (7) an der Basis (6, 16) des Verankerungselements zum Befestigen eines Zahnersatzes oder einer Zahnbrücke umfasst, **dadurch gekenntzeichnet, dass** der zweite Fixierungsabschnitt (5, 15, 32, 36) die Form eines Kegelstumpfes aufweist, dessen kleinere Basis in Nachbarschaft zu dem Zwischenabschnitt (4, 14, 38) angeordnet ist und dessen größere Basis die Basis (6, 16) des Verankerungselements bildet.

2. Verankerungselement nach einem der vorhergehenden Ansprüche, **dadurch gekennzeichnet, dass** der erste Fixierungsabschnitt (3, 13, 30, 34) die Form eines Kegelstumpfes aufweist, dessen kleinere Basis in Nachbarschaft zu dem Scheitel (1, 12) angeordnet ist und dessen größere Basis in Nachbarschaft zu dem Zwischenabschnitt (4, 14, 38) angeordnet ist.

3. Verankerungselement nach einem der vorhergehenden Ansprüche, **dadurch gekennzeichnet, dass** der erste und zweite Fixierungsabschnitt (3, 13, 30, 34; 5, 15, 32, 36) und der Zwischenabschnitt (4, 14, 38) zusammen die Form eines Kegelstumpfes aufweisen, dessen kleinere Basis in Nachbarschaft zu dem Scheitel (1, 12) angeordnet ist und dessen größere Basis die Basis (6, 16) des Verankerungselements bildet.

4. Verankerungselement nach einem der vorhergehenden Ansprüche, **dadurch gekennzeichnet, dass** der erste Fixierungsabschnitt (3, 13, 30, 34) ein Gewinde aufweist.

5. Verankerungselement nach einem der vorhergehenden Ansprüche, **dadurch gekennzeichnet, dass** der zweite Fixierungsabschnitt (5, 15, 32, 36) ein Gewinde aufweist,

6. Verankerungselement nach Anspruch 5, **dadurch gekennzeichnet, dass** der zweite Fixierungsabschnitt (5, 15, 32, 36) mindestens eine Ausnehmung (10, 17, 18) quer zu den Gewindewindungen umfasst, welche an dem Zwischenabschnitt (4, 14, 38) angeordnet ist und eine Kante (11, 19, 20) quer zu den Gewindewindungen umfasst, wobei die Kante (11, 19, 20) eine Schneideeinheit bereitstellt, die eine Nut bildet,

7. Verankerungselement nach Anspruch 5 oder 6, **dadurch gekennzeichnet, dass** der Zwischenabschnitt (4, 14, 38) eine glatte Außenfläche aufweist.

8. Verankerungselement nach Anspruch 7, **dadurch gekennzeichnet, dass** die Außenfläche des Zwischenabschnitts (4, 14, 38), die Fläche, die durch die Täler (8, 22) der Gewindewindungen des ersten Fixierungsabschnitts (3, 13, 30, 34) begrenzt ist, und die Fläche, die durch die Täler (9, 23) der Gewindewindungen des zweiten Fixierungsabschnitts (5, 15, 32, 36) begrenzt ist, Abschnitte der Mantelfläche desselben Kegelstumpfes sind.

## Revendications

1. Élément d'ancrage destiné à être fixé dans un premier tissu osseux situé dans l'os zygomatique (31, 35) et dans un deuxième tissu osseux situé dans le maxillaire (33, 37), les premier et deuxième tissus osseux étant séparés par du tissu non osseux, l'élément d'ancrage ayant une forme allongée et comprenant un sommet (1, 12) et une première partie de fixation (3, 13, 30, 34) disposée au sommet (1, 12) adaptés l'un et l'autre pour la fixation dans le premier tissu osseux, une base (6, 16), une deuxième partie de fixation (5, 15, 32, 36) disposée au niveau de ladite base (6, 16) et adaptée pour la fixation dans le deuxième tissu osseux, une partie intermédiaire (4, 14, 38) qui peut être positionnée dans le tissu non osseux qui sépare les premier et deuxième tissus osseux, et des moyens de fixation (7) au niveau de la base (6, 16) de l'élément d'ancrage pour fixer une prothèse ou un bridge dentaire, **caractérisé en ce que** la deuxième partie de fixation (5, 15, 32, 36) a la forme d'un tronc de cône dont la petite base est adjacente à la partie intermédiaire (4, 14, 38) et la grande base forme la base (6, 16) de l'élément d'ancrage.

2. Élément d'ancrage selon la revendication 1, **caractérisé en ce que** la première partie de fixation (3, 13, 30, 34) a la forme d'un tronc de cône dont la petite base est adjacente au sommet (1, 12) et la grande base adjacente à la partie intermédiaire (4, 14, 38).

3. Élément d'ancrage selon l'une quelconque des revendications précédentes, **caractérisé en ce que** les première et deuxième parties de fixation (3, 13, 30, 34 ; 5, 15, 32, 36) et la partie intermédiaire (4, 14, 38) prises ensemble ont la forme d'un tronc de cône dont la petite base est adjacente au sommet (1, 12) et la grande base forme la base (6, 16) de l'élément d'ancrage,

4. Élément d'ancrage selon l'une quelconque des revendications précédentes, **caractérisé en ce que** la première partie de fixation (3, 13, 30, 34) est filetée.

5. Élément d'ancrage selon l'une quelconque des revendications précédentes, **caractérisé en ce que** la deuxième partie de fixation (5, 15, 32, 36) est filetée.

6. Élément d'ancrage selon la revendication 5, **caractérisé en ce que** la deuxième partie de fixation (5, 15, 32, 36) comprend au moins un creux (10, 17, 18) perpendiculaire aux filets et disposé au niveau de la partie intermédiaire (4, 14, 38), comprenant un bord distinct (11, 19, 20) perpendiculaire aux filets, lequel bord (11, 19, 20) constitue un taillant de rainurage.

7. Élément d'ancrage selon la revendication 5 ou la revendication 6, **caractérisé en ce que** la partie intermédiaire (4, 14, 38) possède une surface extérieure lisse.

8. Élément d'ancrage selon la revendication 7, **caractérisé en ce que** la surface extérieure de la partie intermédiaire (4, 14, 38), la surface délimitée par les fonds de filets (8, 22) de la première partie de fixation filetée (3, 13, 30, 34) et la surface délimitée par les fonds de filets (9, 23) de la deuxième partie de fixation filetée (5, 15, 32, 36) sont des parties de la surface d'enveloppe du même tronc de cône.
